Europäisches Patentamt

European Patent Office

Office européen des brevets

(19)

(11) **EP 1 364 658 A1**

(12) **EUROPEAN PATENT APPLICATION**

(43) Date of publication:
26.11.2003 Bulletin 2003/48

(51) Int Cl.7: **A61K 38/24**, A61P 15/08

(21) Application number: **02100560.8**

(22) Date of filing: **24.05.2002**

| | |
|---|---|
| (84) Designated Contracting States:<br>**AT BE CH CY DE DK ES FI FR GB GR IE IT LI LU**<br>**MC NL PT SE TR**<br>Designated Extension States:<br>**AL LT LV MK RO SI**<br><br>(71) Applicant: **Applied Research Systems ARS**<br>**Holding N.V.**<br>**Curacao (AN)**<br><br>(72) Inventors:<br>• **Loumaye, Ernest**<br>**F-74140 Massongy (FR)** | • **Engrand, Patrick**<br>**1203 Geneve (CH)**<br>• **Arguinzoniz, Miguel**<br>**4123 Allschwil (CH)**<br>• **De Moustier, Beatrice**<br>**1206 Geneva (CH)**<br><br>(74) Representative: **Serono International S.A.**<br>**12, Chemin des Aulx**<br>**1228 Plan-les-Ouates (CH)** |

(54) **Use of varying ratios of gonadotrophins in controlled ovarian hyperstimulation**

(57) The invention provides a use of FSH and LH in the preparation of a medicament for the treatment of infertility in patients aged 38 years and older, wherein the ratio of FSH:LH is at or about 2:1 or greater.

EP 1 364 658 A1

## Description

### Field of Invention

The invention relates to the field of assisted reproductive technologies (ART), such as controlled ovarian hyperstimulation (COH) for *in vitro* fertilisation (IVF), using gonadotrophins for stimulation of follicular development.

### Background of the Invention

Treatment of infertility by assisted reproductive technologies (ART) such as *in vitro* fertilisation (IVF) or intracytoplasmic sperm injection (ICSI), Gamete Intrafallopian Transfer Procedure (GIFT), and Zygote Intrafallopian Transfer Procedure (ZIFT) requires controlled ovarian hyperstimulation (COH) to increase the number of female gametes[1]. Standard regimens for COH include a down-regulation phase in which endogenous luteinising hormone (LH) is suppressed by administration of a gonadotrophin releasing hormone (GnRH) agonist starting in the luteal phase of a menstrual cycle (usually on about day 20 of a menstrual cycle). Suppression of ovarian function usually takes from 8 to 21 days with a GnRH agonist, and may be monitored by monitoring LH or oestradiol levels (LH < 5 IU/L, $E_2$ < 50 pg/ml generally indicate adequate suppression). Down regulation is followed by a stimulatory phase in which follicular development (folliculogenesis) is induced by daily administration of follicle stimulating hormone (FSH), usually at about 75-450 IU/day.

Alternatively, a GnRH antagonist may be used, in which case ovarian stimulation with FSH is started, usually on day 1, 2 or 3 after spontaneous or induced menstruation, and endogenous LH production is suppressed by administration of a GnRH-antagonist starting on about day 6 after menses.

In COH protocols for ART, multiple follicular development is the desired aim. The ovaries are examined by ultrasound, to detect and measure the developing follicles. When there are at least 3 follicles with a mean diameter greater than 16 mm (preferably one of 18 mm), a single bolus of hCG (5-10,000 IU) is given to trigger ovulation. Oocyte recovery is timed for 36-38 hours after the hCG injection. Oocytes are usually recovered from pre-ovulatory follicles, by aspiration.

The rationale for the use of GnRH agonists and antagonists in this context is the prevention of an untimely LH surge that would cause premature ovulation and follicle luteinisation[2]. GnRH agonist regimens have become the accepted norm in the clinic. It has been found that long regimens (i.e., those started in the mid-luteal phase of the cycle preceding COH, or before) are associated with easier patient scheduling, greater follicle yield, and overall better clinical results.[3] The use of GnRH antagonists is relatively new to the clinic, but is expected to show similar benefits, with the added advantage of a shorter treatment regimen.

The traditional source of FSH is human menopausal gonadotrophin (hMG). This product, isolated from the urine of postmenopausal women, consists of an approximately 1:1 (IU:IU) mixture of FSH and LH. It is also known to use a 2:1 mixture of urinary FSH and LH (for example Pergogreen®) in patients in WHO group I (hypogonadotrophic hypogonadism) and WHO group II anovulation (hypothalamic-pituitary dysfunction resulting in arrested or attenuated gonadal function), and in IVF patients.

The use of hMG enriched with FSH in COH has been reported in US patent no. 4,725,579.[4] On days 3 and 4 of a menstrual cycle, normo-ovulatory women were administered the equivalent of 300 IU of FSH and 150 IU of LH by means of a mixture of hMG and urinary FSH. On day 5 and subsequent days, hMG alone was administered, until administration of an ovulation-triggering dose of hCG. An increase in pregnancy rate is reported, versus a regimen using hMG throughout the stimulation.

In older patients, that is, those aged 38 years and older, the response to FSH and hMG is diminished. This has been reported, for example, by Szamatowicz and Grochowski[5], who examined the outcome of an IVF/ICSI program in relation to patient age, and found that age had a significantly negative effect on stimulation parameters, fertilisation rates and implantation rates. Other studies of COH in older patients confirm these observations[6,7,8].

Traditionally, the approach with older patients has been to increase the starting dose of FSH, and to increase the daily dose as the stimulation continues. This has the considerable disadvantage of exposing the patient to high FSH levels, with the risk of ovarian hyperstimulation, a potentially life-threatening condition. Furthermore, some older patients may fail to respond, even at elevated doses of FSH. Such high doses of FSH also considerably augment the cost of a fertility treatment.

### Summary of the invention

It is an object of the invention to provide an improved method of administration of gonadotrophins for COH for use in older patients.

In a first aspect, the invention provides a use of FSH and LH, for the manufacture of a medicament for use in controlled ovarian hyperstimulation for ART in a female patient aged at or about 38 years or older, wherein the FSH and LH are present in a ratio of at or about 2:1 or greater (FSH:LH; IU:IU).

In a second aspect, the invention provides a use of FSH and LH in controlled ovarian hyperstimulation for ART in a female patient aged at or about 38 years or older, wherein the FSH and LH are present in a ratio of at or about 2:1 or greater (FSH:LH; IU:IU).

In a third aspect, the invention provides a method of controlled ovarian hyperstimulation for ART in a female patient aged at or about 38 years or older, the method comprising administering to the female patient FSH and LH in a ratio of at or about 2:1 or greater (FSH:LH; IU:IU).

In a fourth aspect, the invention provides a kit for use in controlled ovarian hyperstimulation (COH) for ART, the kit comprising FSH and LH in a ratio of at or about 2:1 or greater, and instructions for its use in COH in a female patient aged at or about 38 years or older.

**Detailed description of the invention**

The inventors have surprisingly found that mixtures of FSH:LH in a ratio of greater that at or about 2:1 (IU: IU) are particularly effective in treating older patients for infertility, in particular in COH for assisted reproductive technologies (ART).

For the sake of clarity, the ratios mentioned herein are ratios of IU:IU. It is possible to measure gonadotrophins by mass, in which case the mass ratio of FSH:LH will depend on the specific activity of the FSH and LH preparations. To convert mass to total activity, the following equation is used:

$$\text{total activity} = \text{specific activity} \times \text{mass protein}$$

Where "mass protein" is the protein mass of the gonadotrophin sample.

Older patients, for the purposes of this description, are those women of at least 38 years or older, and preferably 45 years and younger, more preferably 42 years and younger.

ART includes, for example *in vitro* fertilisation (IVF) or IVF in conjunction with intracytoplasmic sperm injection (IVF/ICSI), Gamete Intrafallopian Transfer Procedure (GIFT), and Zygote Intrafallopian Transfer Procedure (ZIFT).

The preferred medicaments of the invention have FSH:LH ratios that fall within the range of at or about 2:1 to at or about 3:1. A particularly preferred FSH:LH ratio is at or about 2:1.

Preferred daily doses of FSH in the medicament for COH in ART are as follows: at or about 50-750 IU FSH, more preferably at or about 150-600 IU FSH, even more preferably at or about 225-525 IU FSH, most preferably at or about 300, 375, 450 or 525 IU FSH. The corresponding amount of LH will depend on the ratio of FSH:LH that is chosen. As mentioned above, preferred FSH:LH ratios fall within the range of at or about 2:1 to at or about 3:1. It is possible to change the dose of FSH during the course of the treatment, while maintaining the FSH:LH ratio at a constant value at or within the values mentioned herein. For example, stimulation might be started with 50 to 150 IU FSH, and then increased by 50, 75 or 150 IU per day, depending on the patient's response. Patient response may be evaluated by ultrasound. It is also possible to vary the ratio of FSH:LH during the course of the treatment, provided the ratio is at or within the values recited herein.

The medicament may be administered daily, or every second day. It is contemplated that FSH administration and LH administration might be on alternate days. Preferably FSH and LH administration are on a daily basis.

The FSH and LH that may be used to prepare the medicaments may be urinary or recombinant. Most preferred are recombinant FSH and LH, because of their high purity and freedom from other human proteins. The use of recombinant FSH and LH also allows the ratio in the medicament to be precisely controlled, since recombinant FSH is free of LH contamination, and recombinant LH is free of FSH contamination (unlike the urinary products).

The desired FSH and LH ratio may be achieved by administering a mixture of FSH and LH, in which the two hormones are formulated as a single mixed preparation.

Also contemplated is the administration of FSH and LH at the preferred ratios by administering separate preparations, one containing FSH as active ingredient and the other containing LH as active ingredient. The separate preparations may be supplied as a kit. When separate preparations are administered, it is contemplated that the administration may be simultaneous, separate or sequential.

It is known that hCG has some LH activity. It is also contemplated to replace a part or all of the LH in the medicament with an equivalent amount of hCG. As a general rule 1 IU of hCG is equivalent to 5-7 IU of LH in the pharmacopoeia Van Hell bioassay[9] and thus equivalent doses of hCG can be calculated by a person skilled in the art.

The FSH and LH, whether present in a single or separate preparations, may be formulated in any form suitable for injection, either intramuscular or subcutaneous injection, preferably subcutaneous injection. One example of a formulation is as a lyophilised powder for reconstitution with water for injection, prior to use. The powder will preferably be in admixture with buffers, such that after reconstitution with water the resulting solution will have a pH that is approximately physiological. A typical buffer is phosphate. The medicament may further comprise stabilisers, such as sucrose. It may further comprise sodium chloride, to render it isotonic with physiological saline.

Alternatively, the FSH and LH may be formulated as solutions for injection. The solution will preferably comprise the excipients and additives listed above for the lyophilised powder.

In one embodiment, FSH and LH are used at the preferred ratios for COH for ART in older female patients, as follows:

The patient is administered a gonadotrophin releasing hormone (GnRH) agonist starting in the luteal phase of a menstrual cycle (usually on about day 20-21 of a menstrual cycle). The GnRH agonist may be administered, for example, as a single injection of 3.75 mg of depot Triptorelin, or daily doses of Buserelin or leuprolide acetate. After approximately 8 to 21 days, suppression of ovarian function is confirmed by monitoring LH and oestradiol levels (LH < 5 IU/L, $E_2$ < 50 pg/ml generally indicate suppression). Stimulation is then started with FSH and LH, according to the preferred ratios of the invention. Stimulation may be started with a daily FSH dose of at or about 150 to 225 IU FSH (and the corresponding amount of LH, depending on the ratio chosen), more preferably at or about 225 IU FSH, and increased daily by 75 IU increments (usually the dose increases are started on or about the 6th day of stimulation, if necessary), depending on the patient's response, as detected with ultrasound. Stimulation with FSH and LH in the preferred ratios recited herein is continued until ultrasound reveals at least 3 follicles with a mean diameter greater than 16 mm (preferably one of 18 mm). This usually takes 10 to 16 days. A single bolus of hCG (5'000 to 10'000 IU hCG) is given to trigger ovulation. Oocyte recovery is timed for 36-38 hours after the hCG injection. Oocytes are usually recovered from pre-ovulatory follicles, by aspiration. The collected oocytes are graded for quality, and depending on their maturity, they may be incubated for from 2 to 36 hours, before insemination. A pre-treated sperm sample is incubated with the oocytes under controlled conditions. After 12 to 18 hours, the oocytes are examined for evidence of fertilisation. Morphologically high quality embryos are selected[10] and transferred to growth medium. Approximately 2 to 3 days after oocyte recovery, fertilised oocytes, i.e. embryos at about the 12-cell stage, are transferred to the uterus. Alternatively, embryos may be transferred at the blastocyst stage.

In another embodiment, FSH and LH are used at the preferred ratios for COH for ART in older female patients, as follows:

Stimulation is started on or about the first day of menses to on or about 3 days after menses, with daily doses of at or about 150 to 225 IU FSH (and the corresponding amount of LH, depending on the ratio chosen), more preferably at or about 225 IU FSH, and increased daily by 75 IU increments (usually the dose increases are started on or about the 6th day of stimulation, if necessary) depending on the patient's response, as detected with ultrasound. On at or about day 6 or 7 after menses a GnRH antagonist is administered, for example Antide, Azaline B, Cetrorelix, Ganirelix or Antarelix, and continued until on or about the day of hCG administration. Stimulation with FSH and LH in the preferred ratios recited herein is continued until ultrasound reveals at least 3 follicles with a mean diameter greater than 16 mm (preferably one of 18 mm). This usually takes 10 to 16 days. A single bolus of hCG (5'000 to 10'000 IU hCG) is given to trigger ovulation. Oocyte recovery, fertilisation and embryo transfer are as described above.

The following example illustrates a preferred embodiment of the invention.

### Example

### Materials and methods:

The following abbreviations are used:

r-hFSH: recombinant human follicle stimulating hormone;
r-hLH: recombinant human luteinising hormone
u-hCG: urinary human chorionic gonadotrophin

### Patient group:

Infertile patients aged between 38 and 42 years, desiring fertility, with serum FSH < 15 IU/L, were included in this trial.

### Regimen:

The patients were administered a GnRH agonist (Buserelin), starting 7-8 days post-ovulation (daily subcutaneous injections of 500 μg) until down-regulation was achieved and confirmed by ultrasound (US) and oestradiol (E2) levels. The Buserelin dose was decreased to 250 μg/day thereafter, until day of hCG. Patients were randomised to receive either r-hFSH : r-hLH ratio 1:0 (control group), r-hFSH : r-hLH ratio 3:1, r-hFSH : r-hLH ratio 2:1 or r-hFSH : r-hLH ratio 1:1.

The starting dose of r-hFSH (Gonal-F, Laboratories Serono Int., Geneva, Switzerland) was 225 IU and could be increased by increments of 75 IU from stimulation day 7 onwards and up to a maximum of 450 IU while keeping the ratio of FSH:LH fixed throughout the stimulation period. Serum E2 and US were used to monitor the patients' response.

Criteria for the u-hCG bolus injection (5'000 IU, Profasi, Laboratories Serono Inter., Geneva, Switzerland) were the presence of at least one follicle ≥18 mm and serum E2 levels within an acceptable range.

Oocytes were collected by aspiration, approximately 36 hours after the hCG bolus. This time is termed "ovum pick up", or OPU.

Transfer was limited to 3 embryos. Luteal phase was supported by vaginal progesterone starting on the day of OPU and continuing up to negative pregnancy test or 12 weeks in pregnant patients.

Statistical analyses were performed two-sided at the 0.05 significance level. The mean values obtained among the 4 dose-ratio groups were compared by means of an analysis of variance or covariance. Response rates based on binary endpoints were compared between the treatment groups using logistic regression models or Cochran-Mantel-Haenszel (CMH) statistic.

**Primary efficacy endpoints:**

Primary efficacy endpoints included the number of oocytes retrieved, the implantation rate, the biochemical and clinical pregnancy rates.

**Results:**

One hundred and eighty-nine (189) patients were enrolled and 173 started down-regulation with Buserelin. One hundred and sixty-nine (169) patients were randomised and all of them were used for the efficacy and safety analysis. A similar distribution across the 4 groups was observed (41 to 45 patients received one of the 4 r-hFSH : r-hLH dose-ratios ) which resulted in a similar proportion of patients having had embryos transferred (83 to 90%). The mean age was 39.3 years old (SD: 1.3; range: 37-43). There were no significant differences between treatment groups in terms of mean age, body mass index, type and cause of infertility, serum LH levels on the first day of stimulation. No differences in the number of treatment days and the number of r-hFSH vials used were observed.

The number of cleaved embryos obtained was higher in the 2:1 dose-ratio group than in the 1:1 dose-ratio group (p=0.030). More favourable results were consistently obtained in the 2:1 dose-ratio group than in the 3 other dose-ratio groups in terms of duration of stimulation, number of follicles > 11 mm on day of hCG, number of oocytes retrieved, number of two pronuclei (2 PN) oocytes and viable embryos. Serum mean adjusted E2 levels on day of hCG were found to further increase with the dose of LH administered. However, they were lower in women who received similar amounts of r-hFSH and r-hLH (NS). Serum mean adjusted inhibin B levels on day of hCG were also lower in the 1:1 dose-ratio group in comparison to the 3 other dose-ratio groups. These data combined with the above ovarian response data provide some evidence of an unfavourable effect of the 1:1 dose-ratio on late follicular development.

There was a significant trend (p=0.04) in terms of overall pregnancy rates (i.e. both clinical and biochemical) from the 3:1 (24.3%) to the 2:1 (16.0%) and the 1:1 (7.1%) dose-ratio groups. Total pregnancy rate was significantly higher in the 3:1 dose-ratio group than in the 1:0 dose-ratio group and the 1:1 dose-ratio group (p= 0.030 and 0.043, respectively). Pregnancy rate and clinical pregnancy rate of the pooled 3:1 and 2:1 dose-ratio groups were significantly higher than the pooled 1:0 and 1:1 dose-ratio groups (p= 0.016 and 0.049, respectively). More biochemical pregnancies occurred in the 3:1 dose-ratio group than in the 2:0 dose-ratio group. The 3:1 and 2:1 dose-ratio groups had similar clinical pregnancy rate (12.2.% and 12.0%, respectively) even though the 3:1 had a higher pregnancy rate than the 2:1 dose-ratio group (NS).

No difference in the incidence or intensity of adverse events was observed. Ovarian hyperstimulation syndrome incidence was low and strictly comparable in the 3:1 and 2:1 dose-ratio groups (2.4 %). Local tolerance of r-hLH injections was similar across the 4 treatment groups.

Some parameters are summarised in Table 1.

Table 1.

| Main parameters associated with stimulation with different ratios of FSH:LH | | | | |
|---|---|---|---|---|
| | FSH:LH | FSH:LH | FSH:LH | FSH:LH |
| | 3:1 | 2:1 | 1:1 | 1:0 |

# EP 1 364 658 A1

Table 1. (continued)

| Main parameters associated with stimulation with different ratios of FSH:LH | | | | |
|---|---|---|---|---|
| | FSH:LH | FSH:LH | FSH:LH | FSH:LH |
| Adjusted mean number of follicles ≥ 11 mm on day of hCG | 9.18 | 10.70 | 8.72 | 9.26 |
| Adjusted mean no. of oocytes retrieved | 7.52 | 8.39 | 7.43 | 8.41 |
| Adjusted mean number of viable embryos | 3.71 | 3.67 | 3.00 | 3.38 |
| Adjusted mean implantation rate | 7.29% | 6.78% | 3.38% | 3.42% |
| Adjusted total pregnancy rate | 24.3% | 16.0% | 7.1% | 6.3% |
| Adjusted clinical Pregnancy rate | 12.2% | 12.0% | 2.2% | 4.8% |

**Conclusions:**

The results show that the total and clinical pregnancy rates for the pooled 3:1 and 2:1 dose-ratio groups are significantly higher than those observed in the 1:0 and 1:1 dose-ratio groups. Clinical pregnancy rates are those expected in this age range of patients. These data demonstrate that the addition of r-hLH to r-hFSH has a beneficial effect in ART patients aged between 38 and 42 for enhancing reproductive success. The safety profile is similar across the 4 treatment groups.

**References:**

[1] Healy *et al; Lancet* **343** *1994;* 1539-1544

[2] Filicori, M.; J. *Clin. Endocrinol. Metab.* **81** *1996;* 2413-6

[3] Filicori, M. *et al.; Fertil. Steril.* **65** *1996*; 387-93

[4] US patent no. 4,725,579; Serono Laboratories Inc.; granted February 16, 1988

[5] Szamatowicz & Grochowski; *Fertility and infertility in aging women; Gynecol. Endocrinol.* **12** *1998*; 407-13

[6] Rosenwaks *et al. ; The role of maternal age in assisted reproduction; Hum. Reprod.* **Suppl 1** *1995*; 165-73

[7] Spandorfer *et al.; Effect of parental age on fertilisation and pregnancy characteristics in couples treated by intracytoplasmic sperm injection; Hum. Reprod.* **13** *1998*; 334-8

[8] Marcus *et al. ; In vitro fertilization and embryo transfer in women aged 40 years and over, Human Reproduction* **2** *1996*; 459-468

[9] Van Hell *et al; Acta Endocrin,* **47** *1964;* 409-418

[10] Owen K. Davis and Zev Rosenwaks; "In vitro *fertilisation"* in Reproductive Endocrinology, Surgery, and Technology, Volume II; Chapter 124, pages 2328-2329; Eli Y. Adashi, John A. Rock and Zev Rosenwaks, Eds.; Lippencott-Raven Publishers, 1996, Philadelphia, Penn.

**Claims**

1.  Use of FSH and LH, for the manufacture of a medicament for use in controlled ovarian hyperstimulation for ART in a female patient aged at or about 38 years or older, wherein the FSH and LH are present in a ratio of at or about 2:1 or greater (FSH:LH; IU:IU).

2.  Use according to claim 1, wherein the ART is selected from *in vitro* fertilisation, gamete intrafallopian transfer procedure (GIFT), zygote intrafallopian transfer procedure (ZIFT) and intracytoplasmic sperm injection (ICSI).

3.  Use according to claim 1 or 2, wherein the patient is not older than at or about 45 years old.

4.  Use according to claim 1, 2 or 3, wherein the patient is not older than at or about 42 years old.

5.  Use according to any one preceding claim, wherein the ratio of FSH:LH (IU: IU) is within the range of between at or about 2:1 to at or about 3:1.

**6.** Use according to any one preceding claim, wherein the ratio of FSH:LH (IU:IU) is at or about 2:1.

**7.** Use according to any one preceding claim, wherein the ratio of FSH:LH (IU: IU) is at or about 3:1.

**8.** Use according to any one preceding claim, wherein the medicament is to be administered in daily doses.

**9.** Use according to any one preceding claim, wherein the medicament is to be administered every second day.

**10.** Use according to any one of claims 1 to 9, wherein the medicament is to be administered in daily doses of from at or about 50 IU FSH to at or about 750 IU FSH.

**11.** Use according to any one of claims 1 to 9, wherein the medicament is to be administered in daily doses of from at or about 150 IU FSH to at or about 600 IU FSH.

**12.** Use according to any one of claims 1 to 9, wherein the medicament is to be administered in daily doses of from at or about 225 IU FSH to at or about 525 IUFSH.

**13.** Use according to any one of claims 1 to 9, wherein the medicament is to be administered in daily doses of at or about 300, 375, 450 or 525 IU FSH.

**14.** Use according to any one preceding claim, wherein the FSH and LH are administered simultaneously, separately or sequentially.

**15.** Use according to any one preceding claim, wherein the FSH and LH are present in a single pharmaceutical composition.

**16.** Use according to any one of claims 1 to 14, wherein the FSH and LH are used in separate pharmaceutical compositions.

**17.** Use according to any one preceding claim, wherein the FSH is urinary FSH.

**18.** Use according to any one of claims 1 to 16, wherein the FSH is recombinant FSH.

**19.** Use according to any one preceding claim, wherein the LH is urinary LH.

**20.** Use according to any one of claims 1 to 18, wherein the LH is recombinant LH.

**21.** Use according to any one preceding claim, wherein the LH is replaced in part by an equivalent dose of hCG.

**22.** Use according to any one preceding claim, wherein the medicament is used in combination with a GnRH agonist.

**23.** Use according to any one of claims 1 to 21, wherein the medicament is used in combination with a GnRH antagonist.

**24.** Use of FSH and hCG, for the manufacture of a medicament for use in controlled ovarian hyperstimulation for ART in a female patient aged at or about 38 years or older, wherein the FSH and hCG are present in amounts bioequivalent to a ratio of FSH:LH of at or about 2:1 or greater (FSH:LH; IU:IU).

**25.** Use according to claim 24, wherein the patient is not older than at or about 45 years old.

**26.** Use according to claim 24, wherein the patient is not older than at or about 42 years old.

**27.** A kit for use in controlled ovarian hyperstimulation for *in vitro* fertilisation, the kit comprising FSH and LH in a ratio of at or about 2:1 or greater, and instructions for its use in controlled ovarian hyperstimulation in a female patient aged at or about 38 years or older.

**European Patent Office**

**EUROPEAN SEARCH REPORT**

Application Number

EP 02 10 0560

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | J.BALASCH E.A.: "The effect of exogenous luteinizing hormone (LH) on oocyte viability: evidence from a comparative study using recombinant human follicle-stimulating hormone (FSH) alone or in combination with recombinant LU for ovarian stimulation in pituitary-suppressed women undergoing assisted reproduction" JOURNAL OF ASSISTED REPRODUCTION AND GENETICS, vol. 18, no. 5, 2001, pages 250-256, XP001057073 * page 250 * * page 251 * * page 253, column 2 * * page 254, column 1 * --- | 1-4, 8-15,18, 20-22, 24-27 | A61K38/24 A61P15/08 |
| X | THE EUROPEAN RECOMBINANT HUMAN LH STUDY GROUP: "Recombinant human luteinizing hormone (LH) to support recombinant human follicle-stimulating hormone (FSH)-induced follicular development in LH- and FSH-deficient anovulatory women: a dose-finding study" JOURNAL OF CLINICAL ENDOCRINOLOGY AND METABOLISM, vol. 83, no. 5, 1998, pages 1507-1514, XP000891973 * page 1507 * * page 1508, column 1 * --- -/-- | 1-8,10, 14,15, 18,20,27 | **TECHNICAL FIELDS SEARCHED** (Int.Cl.7) A61K |

The present search report has been drawn up for all claims

| Place of search | Date of completion of the search | Examiner |
|---|---|---|
| THE HAGUE | 15 October 2002 | Peeters, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 02 10 0560

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | E.KOUSTA E.A.: "Successful induction of ovulation and completed pregnancy using recombinant human luteinizing hormone and follicle stimulating hormone in a woman with Kallmann's syndrome" HUMAN REPRODUCTION, vol. 11, no. 1, 1996, pages 70-71, XP001056924 * page 70 * * page 71, column 2 * | 1-6,8, 10,14, 16,18, 20,27 | |
| X | U.D.GORDON E.A.: "A randomized prospective assessor-blind evaluation of luteinizing hormone dosage and in vitro fertilization outcome" FERTILITY AND STERILITY, vol. 75, no. 2, 2001, pages 324-331, XP002199282 * page 324 * * page 325, column 1 * * page 330, column 1 * | 1-8,10, 14-16, 18,20, 21,23,27 | |
| X | S.BURGUES: "The effectiveness and safety of recombinant human LH to support follicular development induced by recombinant human FSH in WHO group I anovulation: evidence from a multicentre study in Spain" HUMAN REPRODUCTION, vol. 16, no. 12, 2001, pages 2525-2532, XP001057228 * page 2525 * * page 2526, column 2 * | 1-4,6,8, 10,14, 15,18, 20,21, 24-27 | TECHNICAL FIELDS SEARCHED (Int.Cl.7) |

-/--

The present search report has been drawn up for all claims

| Place of search | Date of completion of the search | Examiner |
|---|---|---|
| THE HAGUE | 15 October 2002 | Peeters, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 02 10 0560

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 99 55357 A (ASTA MEDICA) 4 November 1999 (1999-11-04)<br><br>* claims 1,4 *<br>* page 3, line 10-26 *<br>--- | 1,2,14, 15,17, 18,20,27 | |
| X | M.FAWZY E.A.: "The effect of gonadotrophins with differing LH/FSH ratios on the secretion of the various species of inhibin in women receiving IVF" HUMAN REPRODUCTION, vol. 16, no. 6, 2001, pages 1092-1097, XP008009343<br>* page 1092 *<br>----- | 1-4,7, 10,14, 17,19,27 | |
| | | | TECHNICAL FIELDS SEARCHED (Int.Cl.7) |

The present search report has been drawn up for all claims

| Place of search | Date of completion of the search | Examiner |
|---|---|---|
| THE HAGUE | 15 October 2002 | Peeters, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 02 10 0560

This annex lists the patent family members relating to the patent documents cited in the above-mentioned European search report.
The members are as contained in the European Patent Office EDP file on
The European Patent Office is in no way liable for these particulars which are merely given for the purpose of information.

15-10-2002

| Patent document cited in search report | Publication date | Patent family member(s) | Publication date |
|---|---|---|---|
| WO 9955357 A | 04-11-1999 | AU 752415 B2 | 19-09-2002 |
| | | AU 3702899 A | 16-11-1999 |
| | | BR 9909802 A | 26-12-2000 |
| | | CA 2330131 A1 | 04-11-1999 |
| | | CZ 20003766 A3 | 15-08-2001 |
| | | WO 9955357 A1 | 04-11-1999 |
| | | EP 1082129 A1 | 14-03-2001 |
| | | HU 0101528 A2 | 28-11-2001 |
| | | JP 2002512975 T | 08-05-2002 |
| | | NO 20005145 A | 13-10-2000 |
| | | PL 343609 A1 | 27-08-2001 |
| | | SK 15302000 A3 | 08-10-2001 |
| | | TR 200003063 T2 | 21-02-2001 |
| | | US 6319192 B1 | 20-11-2001 |

EPO FORM P0459

For more details about this annex : see Official Journal of the European Patent Office, No. 12/82